Europäisches Patentamt

European Patent Office

Office européen des brevets

(11) Veröffentlichungsnummer: **0 567 816 A1**

# (12) EUROPÄISCHE PATENTANMELDUNG

(21) Anmeldenummer: **93105801.0**

(51) Int. Cl.5: **A61K 37/64**

(22) Anmeldetag: **08.04.93**

(30) Priorität: **30.04.92 DE 4214215**

(43) Veröffentlichungstag der Anmeldung:
**03.11.93 Patentblatt 93/44**

(84) Benannte Vertragsstaaten:
**AT BE CH DE DK ES FR GB GR IE IT LI LU NL PT SE**

(71) Anmelder: **BEHRINGWERKE Aktiengesellschaft**
**Postfach 1140**
**D-35001 Marburg(DE)**

(72) Erfinder: **Schüler, Eckhard**
**Albert-Demmitz-Weg 6**
**W-3550 Marburg(DE)**
Erfinder: **Römisch, Jürgen**
**Sonnenhang 13**
**W-3550 Marburg(DE)**
Erfinder: **Pâques, Eric-Paul**
**Auf der Kanzel 4**
**W-3550 Marburg-Haddamshausen(DE)**
Erfinder: **Dickneite, Gerhard**
**Zum Neuen Hieb 31**
**W-3550 Marburg(DE)**

(54) **Verwendung von Inhibitoren von Plasminogenaktivatoren zur Behandlung von Entzündungen und Wunden.**

(57) Die Erfindung betrifft Inhibitoren von Plasminogenaktivatoren, wie Urokinase, uPA oder tPA zur intra- und postoperativen Therapie, zur Behandlung von Verletzungen sowie zur Behandlung und Prophylaxe entzündlicher Erkrankungen. Geeignete Inhibitoren sind insbesondere PAI-1 und PAI-2.

EP 0 567 816 A1

Bei verschiedenen Erkrankungen ist die Regulation der Plasmin-Aktivität gestört. Plasmin wird aus Plasminogen durch Plasminogenaktivatoren (PA) wie z.B. uPA (urinärer PA) oder Gewebsplasminogenaktivator (tPA) gebildet. Die PA's werden durch Plasminogenaktivator-Inhibitoren (PAI) inhibiert. Als physiologisch relevante PAI's sind zur Zeit zwei verschiedene Typen bekannt: Plasminogenaktivator-Inhibitor Typ 1 (PAI-1) und Plasminogenaktivator-Inhibitor Typ 2 (PAI-2). Beide PAI's sind Inhibitoren vom Serpin-Typ.

Im allgemeinen kommt es bei Entzündungen der Augen, der Ohren oder der Haut, z.B. bei einer Schädigung der Cornea, zu einer Erhöhung der Plasminaktivität in der Tränenflüssigkeit. Plasmin wiederum wird für den Abbau von Fibronektin in der extrazellulären Matrix, für eine Förderung der Angiogenese und für eine Aktivierung der Prokollagenase zu aktiver Kollagenase, was den Abbau von Kollagen-Molekülen zur Folge hat, verantwortlich gemacht (Barlati et al. Exp. Eye Res. 51, 1-9 (1990)). Für die Behandlung verschiedener pathologischer Zustände, in denen erhöhte Plasmin-Aktivitäten eine Rolle spielen, wurden bereits niedermolekulare Substanzen wie 2-Aminocapronsäure oder Tranexamsäure (Trans-4-aminoethyl-cyclohexancarbonsäure) eingesetzt. So erwies sich z.B. die Tranexamsäure für die Behandlung von Osteoarthritis geeignet. Ebenso konnte die bei Colitis ulcerosa und verwandten Erkrankungen überschießende fibrinolytische Aktivität mit Hilfe von Tranexamsäure reduziert werden. Die oben genannten niedermolekularen Plasmin-Inhibitoren sind jedoch auf Grund ihrer toxischen Nebenwirkungen von Nachteil.

Mit Aprotinin steht ein weiterer Plasmin-Inhibitor zur Verfügung. Er erwies sich für eine Behandlung von Corneal ulcer als bedingt geeignet.

Es wurde nun überraschenderweise gefunden, daß Entzündungen insbesondere der Augen, der Ohren und/oder der Haut aber auch beispielsweise Osteoarthritis oder Colitis ulcerosa auf vorteilhafte Weise mit Inhibitoren von Plasminogenaktivatoren behandelt werden können.

Gegenstand der Erfindung ist daher die Verwendung von Inhibitoren von Plasminogenaktivatoren zur Herstellung eines Arzneimittels zur Therapie und/oder Prophylaxe von Entzündungen der Augen, der Ohren, der Haut, von Osteoarthritis und/oder von Colitis ulcerosa.

Gehemmt werden insbesondere Urokinase (uPA) und/oder tPA, vor allem uPA und/oder tPA. Geeignete Inhibitoren sind insbesondere PAI-1 und/oder PAI-2 und deren Mutanten oder Varianten, vor allem PAI-2, insbesondere Fragmente von PAI-2, beispielweise Deletionen am N-Terminus vor allem der N-terminalen Aminosäuren 1-82 bzw. 1-84 von reifem PAI-2. Die Inhibitoren können entweder allein oder in Kombination mit anderen Medikamenten, z.B. Antibiotika, verwendet werden, wobei sie sowohl lokal als auch systemisch appliziert werden können. Bei Osteoarthritis werden die Wirkstoffe vorzugsweise intraartikulär oder intraoperativ (offen oder arthroskopisch) appliziert. Eine wirksame Dosis liegt im allgemeinen für eine topische Anwendung bei 1 - 300 000 Urokinase-inhibierenden Einheiten und vorzugsweise bei 500 - 50 000 Urokinase-inhibierenden Einheiten, für eine i.v. - oder für eine lösungsvermittelte i.m.- Applikation bei 50 - 750 000 Urokinase-inhibierenden Einheiten pro kg Körpergewicht.

Die Inhibitoren, vor allem PAI-2, können durch Zusatz von physiologisch verträglichen Stabilisatoren wie Albumin, Polygelin, Gelatinehydrolysat, Glycin, Cystein, Glucose, Lactose, Maltose und/oder Saccharose stabilisiert werden.

Anwendungsgebiete der Inhibitoren sind im allgemeinen die lokale Therapie und/oder Prophylaxe von entzündlichen Augenerkrankungen wie Corneal ulcer, Uveitis, Konjunktivitis, bei intra- und postoperativer Behandlung, zum Schutz der Augenstruktur, vor allem bei entzündlichem Verlauf, von entzündlichen Ohrenerkrankungen wie z.B. Mittelohrentzündung, Trommelfellentzündung, Trommelfellriß mit entzündlichem Verlauf, aber auch von Hauterkrankungen mit Entzündungserscheinungen wie Rötung, insbesondere bei der Infiltration von Gewebe durch Entzündungszellen, wie Makrophagen, Monozyten und Granulozyten, Acantholyse, Bläschenbildung wie z.B. bei Pemphigus, Ekzem, Kontaktdermatitis und atopischer Dermatitis, Verbrennungen mit entzündlichem Verlauf, Vaskulitis, die Förderung der Wundheilung, die Therapie von offenen Wunden und dermalen Ulcera. Weitere Anwendungsgebiete sind die Behandlung epithelialer Läsionen, Osteoarthritis, Colitis ulcerosa, Morbus Crohn, IBD (Inflammatory bowel disease), Pankreatitis, Akne, Behandlung epithelialer Läsionen, cornealer Läsionen, Narbenbildung bei Augenverletzungen, Skleritis, nicht-heilender Cornea-Erosionen, Xerose, Keratose. Des weiteren eignen sich die Inhibitoren allein oder in Kombination mit weiteren Wirkstoffen vorzugsweise als Spüllösungen bei der intra- und postoperativen Behandlung und/oder bei der Behandlung von offenen Wunden der genannten Entzündungen. Eine weitere vorteilhafte Verwendungsart ist die Behandlung von Kontaktlinsen vor dem Tragen mit einer Lösung der genannten Inhibitoren allein oder in Kombination mit weiteren Wirkstoffen zur Hemmung der Neovaskularisierung, zur Therapie von Augenverletzungen und von Augenentzündungen und/oder zur Behandlung von cornealen Infiltraten der Augen und von Hornhautentzündungen der Augen von Kontaktlinsenträgern.

Im folgenden werden einige bevorzugte Verwendungen von Inhibitoren von Plasminogenaktivatoren, wie z.B. PAI-2 im einzelnen beschrieben:

1. Die Verwendung bei der Behandlung nach Verletzung der Augen-Hornhaut durch Laserkeratektomie, aber auch durch Keratotomie. Dieses Laserkeratektomie-Verfahren kann Anwendung finden bei der Korrektur des Brechungsindex bei Kurz- und Weitsichtigen, zur Korrektur von Astigmatismus, zur Korrektur von Hornhautfehlern, zur Entfernung von Narben, zur Keratoplastie, zur Glättung der Hornhaut bei Austrocknungserscheinungen, bei frischen Verletzungen der Hornhaut, bei Infektionen des Auges z.B. durch Viren (z.B. Herpes), Bakterien, Pilze oder Parasiten, bei Kalkeinlagerungen unterhalb der Epithelschicht (eine Alterserscheinung), bei Verwachsungen der Bindehaut mit der Hornhaut (Pterygien).

2. Die Verwendung bei Augenentzündungen und Augentrübungen, z.B. nach Laserbehandlung, normaler Augenchirurgie (z.B. Vitrektomie, Kataraktchirurgie, extrakapsuläre Kataraktextraktion, Linsenoperationen, Linsenaustausch, Linsenimplantation, Keratoplastie, Hornhauttransplantationen), bei Konjunktivitis, Keratokonjunktivitis, Keratokonjunktivitis sicca, Iritis, Iridocyclitis, Keratitis, Graves Ophthalmopathie, Mooren's ulcer, Vaskulitis, Uveitis, bei allergischen Erscheinungen im Auge, Infektionen, Stoffwechselstörungen, Entzündungserkrankungen und Autoimmunerkrankungen (z.B. Systemischem Lupus erythematodes, Wegener Syndrom, rheumatischer Arthritis, Sarcoidose, Polyarthritis, Pemphigus, Pemphigoid, Erythema multiforme, Sjögren's Syndrom, entzündliche Darmerkrankungen (Inflammatory Bowel Disease), Multiple Sklerose, Myasthenia gravis, Keratitis, Skleritis.

3. Die Verwendung zur Vermeidung der Narbenbildung am Auge nach chirurgischem Eingriff bzw. nach Verletzung.

4. Die Verwendung zur Beschleunigung der Wundheilung und optimalen Regeneration (z.B. glatte Grenzfläche zwischen altem Stroma und neu gebildetem Epithel), zur Normalisierung und Stabilisierung der Stoffwechselfunktionen des Epithels und des Stromas nach Verletzung oder chirurgischem Eingriff.

5. Die Verwendung zur Hemmung der Neovaskularisierung bei Retinopathie, insbesondere bei Diabetikern; bei Retinaablösung, Gefäßverletzung der Retina, bei Entzündung der Retina und Uvea, bei Hornhauttransplantationen.

6. Die Verwendung bei Transplantationen am Auge, insbesondere bei Hornhauttransplantationen. PAI-2 kann hier durch eine Reduktion der Anlockung von PMN die Sensibilisierung reduzieren, die Neovaskularisierung verhindern, ohne gleichzeitig die Wundheilung negativ zu beeinflussen. Dies mindert das Abstoßungsrisiko.

7. Die Verwendung bei Ödemen im Augenbereich, z.B. Makula-Ödem, Submakula-Ödem, Ödem nach Photokoagulation, Cornea-Ödem, Konjunktiva-Ödem, Retina-Ödem, Ödem in der Umgebung des Auges.

8. Die Verwendung bei Infiltrationen im Augenbereich, v.a. der Cornea, der Augenkammer, der Konjunktiva und der Sklera.

9. Die Verwendung zur Therapie des Corneal ulcer.

PAI-2 kann beispielsweise wie folgt hergestellt werden: Wie in EP 0 238 275 beschrieben, wurde die PAI-2-cDNA nach dem Fachmann bekannten Verfahren isoliert, kloniert, in Vektoren ligiert und in geeignete Wirtszellen transformiert bzw. transfiziert. Die Zellen wurden nach bekannten Verfahren fermentiert und aufgeschlosssen. Zur Reinigung von rPAI-2 aus z.B. E.coli-Lysaten eignet sich eine Kombination verschiedener Chromatographie-Methoden (z.B. Ionenaustauschchromatographie über Q-sepharose, Hydrophobe Chromatographie über Phenylsepharose, Gelfiltration über Sephacryl oder Fractogel). Zur Reinigung von rPAI-2 aus Hefe-Lysaten eignet sich ein Verfahren, bestehend aus Basisreinigung (Zellabtrennung, Zellaufschluß mittels Kugelmühle, Cross-Flow-Filtration, Cross-Flow-Konzentration) und anschließender Hochreinigung (Chromatographische Verfahren wie z.B. in WO 91/02057 beschrieben oder mittels Ionenaustauschchromatographie und Immunaffinitätschromatographie). Die spezifische Aktivität ist im allgemeinen 150 000 U/mg oder mehr bezogen auf die Hemmung humaner Urokinase.

Die Herstellung von PAI-1 kann auf analoge Weise, beispielsweise gemäß WO 90/13648 hergestellt werden.

Die Vorteile der Inhibitoren von Plasminogenaktivatoren sind vor allem eine im allgemeinen nicht-toxische und nebenwirkungsfreie Monotherapie der genannten Entzündungen bei gleichzeitiger Hemmung der Neovaskularisierung. Da die Hemmung der Plasminogenaktivatoren wesentlich niedrigere Applikationskonzentrationen als bei Plasmininhibitoren erlaubt, ist die gleichzeitige Applikation bzw. die Kombination mit anderen Wirkstoffgruppen wie z.B. Antibiotika besonders vorteilhaft. Ferner ist die applizierbare Menge an Inhibitoren von Plasminogenaktivatoren deutlich geringer als bei Plasmininhibitoren und zudem ist die Gefahr einer allergischen Reaktion im allgemeinen vermindert. Auch wurde eine vorteilhaft lange Wirkungsdauer im Vergleich zu Plasmininhibitoren beobachtet.

**Beispiel 1:**

Anwendung von PAI-2 für die Therapie von Corneal ulcer

In dem von Cejkova at al. 1975 (Histochemistry 45: 71-75) beschriebenen Kaninchen-Modell für Corneal ulcer wurde PAI-2 in verschiedenen Konzentrationen (5-100 $\mu$g/ml) lokal ins Auge appliziert (25 $\mu$g/ml drei mal täglich; 100$\mu$g/ml zwei mal täglich). Die Kontrollen erhielten gleiche Volumina (1 ml) des Lösungsmittels (physiologische Kochsalzlösung, pH 7,2) und wurden zweimal täglich appliziert. Als Referenzsubstanz diente Aprotinin (5000 IU/ml in den ersten zwei Wochen, zweimal täglich; in den folgenden zwei Wochen wurde die Dosis auf 2500 IU/ml reduziert).

Nach der Behandlung mit 0,75 M Natriumhydroxid mit Hilfe eines Plastiktubus von 11 mm Innendurchmesser, kommt es zu einem massiven Influx von Entzündungszellen: Diese sezernieren proteolytische Aktivität und induzieren gleichzeitig die Keratinozyten, auf dem Cornea-Epithel ein anderes Proteoglycan-Spektrum zu synthetisieren. Dies hat Störungen in der Ordnungsstruktur der Cornea zur Folge, was wiederum zu einem Verlust der Transparenz führt.

Gemessen wurden folgende Parameter: Perforation der Cornea, Ulcus- Bildung, Neovaskularisierung, Plasmin-Aktivität in der Tränenflüssigkeit, Entzündungsmerkmale wie Einstrom von Entzündungszellen, Transparenz des Auges, Sichtbarkeit der Iris (Tab. 1). Verglichen mit der Placebo-Gruppe ergaben sich folgende Vorteile bei den PAI-2 -Gruppen: Die Plasmin-Aktivität konnte auf ein Minimum reduziert werden. Um mit Aprotinin das gleiche Ausmaß an Reduktion der Plasmin-Aktivität zu erreichen, muß häufiger und öfter appliziert werden. Mit PAI-2 wurden die Perforation und die Ulcus-Bildung komplett verhindert. Diese Effekte konnten auch mit Aprotinin erreicht werden. Überraschenderweise wurde mit PAI-2 die Anzahl der in das als Folge des experimentellen Reizes einströmenden Entzündungszellen drastisch und nachhaltig reduziert und zwar auf das bei gesundem Gewebe vorgefundene Normalmaß.

Folgende positive Effekte konnten darüberhinaus überraschenderweise erreicht werden: Die für eine vollständige Abheilung unumgänglich notwendige Hemmung der Neovaskularisierung wurde mit PAI-2 bewirkt, so daß die Transparenz des Auges wiederhergestellt werden konnte und die Iris wieder sichtbar wurde. Bei der Behandlung mit Aprotinin wird zwar die Ulceration und die Perforation der Cornea verhindert, die Transparenz jedoch bleibt verloren. Die schon mit relativ niedrigen PAI-2-Dosierungen erreichten positiven Effekte waren auch nicht mit höheren Aprotinin-Dosierungen zu erreichen.

Die Hemmung von Plasminogenaktivatoren durch PAI-2 eröffnet zudem gegenüber der Hemmung von Plasmin, z.B. durch Aprotinin, noch weitere Vorteile: Die ausschließliche Behandlung mit Inhibitoren von Plasminogenaktivatoren ist für einen Behandlungserfolg ausreichend, was einen Vorteil gegenüber der herkömmlichen Therapie darstellt. Die extrem hohe Potenz des verwendeten Wirkprinzips erlaubt die Verwendung sehr geringer Inhibitor-Konzentrationen, so daß auch noch eine Kombinationstherapie, z.B. mit Antibiotika, wie Chloramphenicol, gut möglich ist.

In einem weiteren Versuch wurde die Behandlung des Corneal ulcer nicht direkt nach der Induktion, z.B. mit Alkali-Einwirkung, begonnen sondern erst nach 10 Tagen. Zu diesem späten Zeitpunkt ist die Plasmin-Aktivität in der Tränenflüssigkeit schon beträchtlich auf Werte von 2-2,5 $\mu$g/ml angestiegen.

Beginnt man zum gleichen Zeitpunkt (10 Tage nach der Induktion) mit einer Aprotinin-Behandlung (5000 IU/ml; lokale Applikation zweimal täglich), so geht die in der Tränenflüssigkeit nachweisbare Konzentration an aktivem Plasmin auf Normalwerte zurück. Dieser Effekt hält aber nur für ca. zwei Stunden an, dann steigen die Plasmin-Werte wieder an.

Beginnt man zu diesem Zeitpunkt mit einer PAI-2-Behandlung (z.B. 100 $\mu$g/ml; lokale Applikation), so fällt die aktive Plasminkonzentration auf Normalwerte (0,4-0,6$\mu$g/ml) zurück. Der Effekt einer einmaligen PAI-2-Applikation hält überraschenderweise sehr lange an. So liegt z.B. die Plasminkonzentration in der Tränenflüssigkeit 48 Stunden nach einer einmaligen PAI-2-Applikation noch bei 1$\mu$g/ml. Hierbei waren alle drei getesteten PAI-2 Konzentrationen (5, 25, 100 $\mu$g/ml) geeignet, den ulcerativen Prozeß zu reduzieren. Die besten Therapieergebnisse wurden bei einer PAI-2-Konzentration von 25 $\mu$g/ml erzielt.

Dieser überraschend lang anhaltende Effekt wird nur bei einer Inhibierung der Plasminogenaktivatoren durch PAI-2 beobachtet, jedoch nicht bei einer Hemmung des Plasmins durch Aprotinin.

Während die lokale Applikation von Steroiden beispielsweise von Dexamethason-natriumphosphat bei der Behandlung des Auges mit Nebenwirkungen, wie z.B. Veränderung der Quellung des Glaskörpers und des Augendruckes, verknüpft ist, zeigt eine Behandlung des Auges mit PAI-2 beim normalen Auge keine Nebenwirkungen. So bleiben Histologie wie z.B. das Muster der lamellären Struktur des anterioren Stroma, aber auch biochemische Befunde wie z.B. die Hydrierung der Cornea, enzymatische Aktivitäten (Laktatdehydrogenase, saure Phosphatase, saure Glycosidase, alkalische Phosphatase, Succinatdehydrogenase), unverändert. Außerdem konnte gezeigt werden, daß neben der erfolgreichen Entzündungshemmung

4

die Reepithelialisierung nicht gehemmt wird.

Messung der Plasminaktivität in der Tränenflüssigkeit:

Kleine Papierscheibchen (Whatman Filterpapier; 5 mm Durchmesser) wurden in einer Substrat-Lösung von D-Val-Leu-Lys-trifluoromethylaminocoumarin (FCA, von Enzymes Systems Products, Livermore, CA, USA) getränkt und getrocknet. Die Substrat-Lösung wurde folgendermaßen präpariert: 1 mg Substrat wurde in 4 Tropfen N,N-dimethylformamid gelöst und 1 ml 0,1 M Tris-HCl pH 7,2 zugegeben. Plasminlösungen mit 0,2-0,5 $\mu$g/ml wurden hergestellt durch Lösen von Plasmin (Sigma) in 0,1 M Tris-HCl-Puffer mit pH 7,2. 20 $\mu$l jeder Konzentration wurden auf die mit Substrat-Lösung vorbehandelten Papierscheibchen getropft und dann in einer feuchten Kammer bei 37°C (in einem Thermostaten), zusammen mit den mit Tränenflüssigkeit getränkten Papierscheibchen inkubiert. Zur Bestimmung der Plasmin-Konzentration in der Tränenflüssigkeit wurden die Papierscheibchen auf die Cornea gelegt und die Tränenflüssigkeit wurde 5 Sekunden lang aufgesaugt. Während der Inkubation der Papierscheibchen bei 37°C wurden diese in 2-Stunden-Intervallen im UV-Licht untersucht. Die Intensität der im UV-Licht emittierten gelben Fluoreszenz der Tränenflüssigkeitsproben wurde mit den Proben mit bekannter Plasminkonzentration (Eichkurve aus Papierscheibchen mit bekannter Plasminkonzentration) verglichen und so die Plasminaktivität der Proben ermittelt.

Tab. 1:

Behandlung von Alkali-induziertem Corneal ulcer

**Testgruppen:**

Gruppe 1: Plazebo-Gruppe (physiologische Kochsalzlösung; wurde zweimal täglich appliziert)

Gruppe 2: Aprotinin-Gruppe (5000 IU/ml zweimal täglich appliziert)

Gruppe 3: rPAI-2 (100 $\mu$g/ml; zweimal täglich appliziert)

Gruppe 4: rPAI-2 ( 25 $\mu$g/ml; dreimal täglich appliziert)

| **Parameter** | **Behandlungsresultat der 4 Testgruppen nach 4 Wochen Behandlung** | | | |
|---|---|---|---|---|
| | **1** | **2** | **3** | **4** |
| Transparenz | − | − | − | + |
| Iris sichtbar | − | − | − | + |
| Entzündung | + | schwach | − | − |
| Erhöhte Plasminaktivität | + | − | − | − |
| Neovaskularisierung | + | + | schwach | − |
| Ulcer-Bildung | + | + | − | − |
| Perforation | + | − | − | − |

**Beispiel 2:**

Verwendung von PAI-2 zur Regeneration des Hornhautepithels

In einem Kaninchen-Modell wurde der Effekt von PAI-2 auf die Regeneration von Hornhautepithel untersucht.

Modellbeschreibung:

Die Untersuchungen wurden mit adulten Chinchilla-Kaninchen (2,5- 3,0 kg Körpergewicht) durchgeführt. Die Tiere wurden anästhesiert durch i.v.-Verabreichung von Thiopental (25 mg/kg Körpergewicht). Die Augen wurden dann fixiert und die Hornhaut mechanisch verletzt: Die ganze Hornhaut wurde vorsichtig zweimal deepithelialisiert (im Abstand von einer Woche) mit Hilfe eines Graefe'schen Messers. Anschließend wurde PAI-2 (in PBS-Puffer, pH 7,2) in verschiedenen Konzentrationen (5-50 $\mu$g/ml) lokal ins Auge appliziert (3 mal täglich). Die Kontrolltiere erhielten gleiche Volumina (0,5 ml) an PBS-Puffer (pH 7,2) oder Aprotinin (60

$\mu$g/ml) oder Flurbiprofen (0,1 % Lösung) in PBS-Puffer, pH 7,2.

Die Tiere wurden nach verschiedenen Zeiten (nach 1, 4, 7, 14, 21, 28 Tagen) nach Setzen der Verletzung getötet. Die Hornhautstruktur wurde histologisch und histochemisch untersucht.

In der Tränenflüssigkeit wurde die Plasmin-Aktivität bestimmt und mit den histologischen Daten korreliert. Zweimal pro Woche wurde das optische Erscheinungsbild der Augen photographisch dokumentiert.

Die Bestimmung der Plasmin-Aktivität erfolgte wie unter Beispiel 1 beschrieben.

Nach der Tötung der Tiere wurden die Augen sofort enukleiert, die Hornhaut oder das ganze anteriore Augensegment ausgeschnitten.

Einige Corneae wurden einer Silberimprägnationstechnik nach McGovern (1955) unterzogen. Nach dem Abziehen des Epithels wurden die Präparationen in Gelatine gebracht. Alle anderen Methoden wurden mit den ganzen anterioren Augensegmenten durchgeführt. Ein Teil wurde gequencht in Leichtbenzin, das mit einer Mischung aus Aceton und Trockeneis gekühlt war. In einem Kryostat wurden Schnitte (12 $\mu$m dick), parallel oder perpendikulär zur Augenoberfläche, durchgeführt. Dann wurden die Schnitte auf nicht vorgekühlte Objektträger oder semipermaeble Membranen überführt und auf Glucosaminoglykane, Aminopeptidase M (APM), Dipeptidylpeptidase IV (DPP IV), Gamma-Glutamyltransferase (GGT), alkalische Phosphatase (AlP), Na$^+$-K$^+$-abhängige Adenosintriphosphatase (ATPase) und Dehydrogenasen (Succinatdehydrogenase SDH, Laktatdehydrogenase LDH) untersucht.

Vor dem Nachweis von APM, DPP IV, GGT und AlP wurden die Schnitte für 2 Minuten fixiert mit einem kaltem Gemisch aus Chloroform und Aceton (1:1). AlP wurde nachgewiesen über eine Azo- Kopplunsmethode unter Verwendung von Naphthol-AS-MX-phosphat (Calbiochem, La Jolla; Ca, USA) und Fast Blue B salt (Michrome, Gurr, Poole, UK). APM wurde nachgewiesen mit Ala-4-methoxy-$\beta$-naphthylamin-Ala-MNA (Bachem, Bubendorf, Schweiz) und Fast Blue B salt (FBB, Michrome Gurr, UK), DPP IV mit Gly-Pro-MNA (Bachem; Schweiz) und Fast Blue B salt (Michrome,Gurr, UK), GGT mit Gamma-Glu-MNA (Bachem, Schweiz), Gly (Lachema, Brno, CSFR) und Fast Blue B nach Lojda et al.(1979). Schnitte auf semipermeablen Membranen wurden verwendet für den Nachweis von saurer Phosphatase (AcP) - mittels Azo-Kopplungsreaktion mit Naphthol-AS-BI-phosphat (Calbiochem) und Hexazonium p-rosanilin, das aus Acridinfreiem rosanilin (Merck, Darmstadt, Germany) hergestellt wurde - und von $\beta$-Glucuronidase ($\beta$-glu) und N-acetyl-$\beta$-D-glucosaminidase (Ac-Glu) - Substrate: Naphthol-AS-BI-$\beta$-glucuronid und Naphthol-AS-BI-N-acetyl-$\beta$-D-glucosaminid (beide von Calbiochem); für beide wurde das Kopplungsreagens Hexazonium-p-rosanilin verwendet. Saure $\beta$-Galaktosidase ($\beta$-Gal) wurde mit Hilfe der indigogenen Methode von Lojda (Cejkova und Lojda, 1975; Lojda et al; 1979) unter Anwendung von 4-Cl-5-Br-3-indolyl-$\beta$-D-galactosid (Cyclo Chemicals, Los Angeles, CA,USA) nachgewiesen.

ATPase wurde nach Cejkova und Lojda (1978) unter Verwendung von ATP-Tris und Ba$^{2+}$-Salz nachgewiesen.

Dehydrogenasen wurden in unfixierten Kryostat-Schnitten nach Lojda et al.(1979) detektiert mit Nitro BT (Lachema,Brno,CSFR) als Elektronenakzeptor.

Für den Nachweis von Glucosaminoglykanen, wurden die Schnitte fixiert in kaltem Ethanol (5 Minuten) und mit einer 1%-igen Alcian blue Lösung, die verschiedene Konzentrationen an MgCl$_2$ enthielt, gefärbt (Cejkova et al., 1973), aber auch mit einer 1%-igen Lösung von Alcian blue in 3%-iger Essigsäure, pH 2,5.

Der verbleibende Teil des anterioren Augensegmentes wurde fixiert in 4 % Paraformaldehyd in 0,1 M Phosphat-Puffer, pH 7,2, und dann in einem Kryostaten geschnitten. 12 $\mu$m-dicke Schnitte wurden auf Albumin-beschichtete Objektträger übertragen, aufgetaut und getrocknet. Fixierte Schnitte wurden für den Nachweis von DPP IV (mit Gly-Arg-MNA, Bachem Schweiz, und Nitrosalicylic aldehyde, NSA, Merck) und DPP II (mit Lys-Pro-MNA und Fast Blue B) verwendet (nach Lojda, 1985 und Cejkova und Lojda 1986).

Einige der fixierten Hornhäute wurden auf morphologische Veränderung (mittels Hämatoxylin-Eosin-Färbung) untersucht.

Puffer-Kontrolle:

Im unbehandelten Auge steigt die Plasmin-Aktivität von 0,2-0,4 $\mu$g/ml innerhalb von 2 Stunden nach der Verletzung an. Nach einem Tag ist eine Plasmin-Aktivität von 1,0-2,0 $\mu$g/ml erreicht, bleibt auf diesem Wert bis zum Tag 7, fällt dann bis zum Tag 14 auf 0,2- 0,4 $\mu$g/ml ab und erreicht nach 21 Tagen einen Wert von kleiner als 0,2 $\mu$g/ml (s. Tab. 2).

Das Epithel regeneriert sich vom Limbus her und enthält verschiedene Enzymaktivitäten (DPP IV, saure Glycosidasen und lysosomale Proteasen (s. Tab. 3)). Innerhalb von 4 Tagen nach der De-epithelialisierung wird ein massiver Einstrom von Entzündungszellen (vor allem polymorphkernige neutrophile Granulozyten, PMN) ins Stroma beobachtet. In der Peripherie findet eine Neovaskularisieung der Hornhaut statt. Einige Keratinozyten werden nekrotisch. Diese werden von Keratinozyten aus der Nachbarschaft ersetzt. Nach 14-21 Tagen ist die Re-epithelialisierung abgeschlossen. Als negative Folgen sind Angiogenese und Transparenzverlust zu nennen (die Transparenz wurde nicht wieder voll hergestellt).

Die aufgetretene Hornhauttrübung war irreversibel.

Behandlung mit Flurbiprofen:

Ähnlich wie bei der Pufferkontroll-Gruppe stieg die Plasmin- Aktivität in der Tränenflüssigkeit an (s. Tab. 2). Im Gegensatz zur Pufferkontrolle strömten weniger Entzündungszellen ins Stroma ein, die Neovaskularisierung war behindert, jedoch nicht blockiert. Bei einigen Augen fand eine Re-epithelialisierung nicht statt, manchmal war sie sogar inhibiert. Das Hornhautepithel zeigte nur sehr niedrige Aktivitäten an ATPase und GGT. Die Daten sind in Tab. 3 zusammengefaßt.

Behandlung mit Aprotinin oder PAI-2:

Während des ganzen Versuchsablaufes waren die Plasmin-Aktivitäten signifikant reduziert. Die Maximalwerte bei der Aprotinin-Gruppe lagen bei 1,0 $\mu$g/ml, in den PAI-2 Gruppen bei nur 0,4 $\mu$g/ml (s. Tab. 2).

Die Aktivitäten von DPP IV, sauren Glycosidasen, lysosomalen Hydrolasen sind in beiden Behandlungsgruppen nur relative schwach, diejenigen von ATPase und von GGT sind normal.

Unter Aprotinin-Behandlung wandern nur relativ wenig Entzündungszellen ins Stroma ein, in den PAI-2-Gruppen sind fast keine mehr nachzuweisen.

Die Transparenz wurde in beiden Behandlungsgruppen wiederhergestellt.

In der Aprotinin-Gruppe war die Wundheilung erst nach 14 Tagen abgeschlossen, während die Wundheilung in den PAI-2 Gruppen schon am Tag 10 nach der De-epithelialisierung wieder abgeschlossen war.

Durch Aprotinin-Behandlung konnte die Neovaskularisierung der Hornhaut nicht verhindert werden, während die Behandlung mit PAI-2 (in allen angewendeten Konzentrationen) die Angiogenese vollkommen blockierte.

Die Vorteile einer Behandlung mit PAI-2 bzw. Inhibitoren von Plasminogenaktivatoren liegen somit in einer Beschleunigung der Re-epithelialisierung, in einer vollständigen Blockierung der Neovaskularisierung, in einer vollständigen und schnellen (schneller als mit Aprotinin oder einer anderen Substanz) Wiederherstellung der Transparenz. PAI-2 unterdrückt die Entzündung der Hornhaut und des Stromas sehr gut. So werden z.B. fast kaum ins Entzündunggsgebiet eingwanderte PMN nachgewiesen. Die enzymatischen Aktivitäten des Hornhautepithels normalisieren sich unter der Behandlung mit PAI-2 sehr schnell wieder. Als zusätzlicher Vorteil ist zu werten, daß die angewendeten PAI-2 Konzentrationen weitaus geringer als die von Aprotinin waren.

PAI-2 war selbst in Konzentrationen von 10 $\mu$g/ml noch voll aktiv. Hiermit ist der Vorteil von PAI-2 verbunden, daß PAI-2 evtl. noch mit anderen Wirkstoffen gleichzeitig appliziert werden kann.

Tab. 2

| PLASMIN ACTIVITY IN THE TEAR FLUID ($\mu$g/ml) | | | | |
|---|---|---|---|---|
| Repeated de-epithelialization | | | | |
| Time (h,d) | Buffer | r-PAI-2 | Aprotinin | Flurbiprofen |
| 2 h | 0.2-0.4 | 0.2-0.4 | < 0.2 | 0.2-0.4 |
| 1 d | 1.0-2.0 | 0.2-0.4 | 0.2-0.4 | 1.0-2.0 |
| 4 d | 1.0-2.0 | 0.2-0.4 | 0.4-1.0 | 1.0-2.0 |
| 7 d | 1.0-2.0 | 0.2-0.4 | 0.2-0.4 | 1.0-2.0 |
| 14 d | 0.2-0.4 | < 0.2 | < 0.2 | 0.2-0.4 |
| 21 d | < 0.2 | | | < 0.2 |

Tab. 3  **Repeated corneal de-epithelisation of the rabbit eye**

| Parameter | Test groups | | | |
| --- | --- | --- | --- | --- |
| | Buffer control | Flurbiprofen [10 mg/ml] | Aprotinin [60 µg/ml] | rPAI-2 [10 µg/ml] |
| 1. Plasmin activity in tear fluid | Increase | Increase | slight Increase | no Increase |
| 2. Enzymatic activities in corneal epithelium | | | | |
| – DPP IV | + | low | slight | slight |
| – Acid glycosidases | + | low | slight | slight |
| – Lysosomal hydrolases | + | low | slight | slight |
| – Na+ - K+ -ATPase | – | low | + | + |
| – GGT | – | low | + | + |
| 3. PMN in stroma | Numerous | low | low | Nearly Absent |
| 4. Reepithelisation after wound healing | Normal (14 days) | Prolonged | Enabled (< 14 days) | Accelerated (10 days) |
| 5. Vascularisation | Pronounced | Inhibited (not blocked) | Pronounced | Blocked |
| 6. Transparency of cornea | Irreversible Cloudy | Partially Cloudy | Restored (nearly) | Restored (early) |

**Beispiel 3:**

PAI-2 zur Behandlung von Augenverletzungen bzw. zur intraund postoperativen Behandlung

Die Experimente wurden wie unter Beispiel 2 beschrieben durchgeführt und ausgewertet. Hierbei wurde jedoch nicht das Epithel abgezogen, sondern die Hornhaut wurde durch einen Skalpellschnitt, der in das Stroma mit einer Tiefe von 60-80 % der Stromadicke eindrang und die ganze Hornhautoberfläche umfaßte (5 mm vom Limbus), verletzt. Nach einem Schnitt in die Hornhaut steigt die Plasmin-Aktivität in der Tränenflüssigkeit relativ rasch (innerhalb von 2 Stunden) an und bleibt über mehrere Tage hinweg auf relativ hohen Werten stehen (s. Tabelle 4).

Schon nach einem Tag wandern Entzündungszellen in das Verwundungsgebiet ein. Hier kann man dann auch in unmittelbarer Nähe erhöhte Aktivitäten an lysosomalen Proteasen und von Glycosidasen nachweisen.

Von Tag 1 bis Tag 7 wandern Epithelialzellen in die Schnittwunde ein. Nach 10 Tagen ist die Schnittwunde mit Epithelzellen wieder aufgefüllt. Die Adhäsion der eingewanderten Zellen zum darunter liegenden Stroma ist jedoch sehr schlecht. Daher findet der eigentliche Wundverschluß eher langsam und schleppend statt. Bei der Wundheilung war die Anordnung der Kollagen-Fibrillen unregelmäßig.

Glycosaminoglykane wurden an der Verwundungsstelle nicht nachgewiesen. Die Aktivitäten von Glykosidasen, vor allem von $\beta$-Galaktosidasen, waren im selben Gebiet erhöht.

Vom makroskopischen Erscheinungsbild her ging teilweise die Transparenz der Hornhaut im Bereich der Schnittwunde vorübergehend - das Maximum lag während der Wundheilungsphase zwischen Tag 4 und Tag 7 - verloren.

Die Hornhaut verheilte mit einem nicht-transparenten Narbengewebe. Die Veränderungen der Hornhaut-Transparenz waren irreversibel. In Tab. 5 sind die Daten zusammengefaßt.

Behandlung mit PAI-2:

Während der gesamten Behandlungszeit war bei den mit PAI-2 behandelten Tieren in der Tränenflüssigkeit nur eine minmale Plasmin-Aktivität nachweisbar. Die maximal erreichte Aktivität lag bei 0,4 $\mu$g/ml.

Im Gegensatz zur Pufferkontroll-Gruppe war bei den mit PAI-2 behandelten Tieren die Entzündung komplett gestoppt. So waren z.B. am Tag 1 in den mit PAI-2 versorgten Augen keine Entzündungszellen in unmittelbarer Nähe der Stroma-Wunde nachweisbar.

Die Keratinozyten wurden relativ früh aktiviert. Die einwandernden Epithelialzellen besaßen nur relativ niedrige Aktivitäten an lysosomalen Hydrolasen, wohingegen die Aktivitäten von GGT, ATPase und SDH wieder sehr früh normalisiert waren.

Schon nach nur 4 Tagen war die Schnittwunde wieder mit Epithelialzellen voll aufgefüllt. Diese adhärierten sehr gut am darunter liegenden Stroma. Schon am Tag 10 war die gesetzte ursprünglich klaffende Schnittwunde wieder vollkommen abgeflacht. Die tiefer liegenden Schichten des Stromas heilten unter Beteiligung (Stoffwechsel- und Zellteilungsaktivität) der benachbarten Keratinozyten.

Nach 14 Tagen war die Wundheilung abgeschlossen. Die neu gebildeten Keratinozyten zeigten eine normale Stoffwechselaktivität (z.B. bzgl. GGT), auch waren keinerlei Hinweise auf eine Entzündung gegeben.

Insgesamt ermöglichte die Behandlung mit PAI-2 eine wesentlich beschleunigte und von der Qualität her wesentlich bessere Wundheilung. Die Wundheilung der Hornhaut ergab ein voll transparentes Gewebe, die Narbenbildung unterblieb vollkommen. Die für den Patienten wichtigen Befunde konnten mit beiden PAI-2 Konzentrationen (10 mg/ml und 20 $\mu$g/ml) erzielt werden.

Die PAI-2 Daten sind in Tabelle 5 zusammengefaßt.

Behandlung mit Aprotinin:

Im Vergleich zur Pufferkontroll-Gruppe war die Plasmin-Konzentration in der Tränenflüssigkeit reduziert (s. Tab. 3).

Die Schnittwunde heilt mit normaler Geschwindigkeit ab (langsamer als mit PAI-2).

Vergleichbar mit dem PAI-2-Effekt war bei den einwandernden Epithelialzellen die Aktivität von lysosomalen Hydrolasen und Glykosidasen reduziert.

Die Adhäsion des neu gebildeten Epithels am Stroma war nicht so stark wie bei den mit PAI-2 behandelten Tieren. Die Schnittwunde heilte langsamer, ergab jedoch ein transparentes Gewebe. Histochemisch war die Wundstelle sehr ähnlich im Vergleich zu derjenigen bei den mit PAI-2 behandeten Tieren.

Puffer-Kontroll-Gruppe:

Bei den Tieren der Puffer-Kontrollgruppe war die Plasmin-Aktivität in der Tränenflüssigkeit über längere Zeit erhöht (s. Tab. 4). Gleichzeitig wird ein starker Einstrom von Entzündungszellen (vorwiegend PMN) in die Hornhaut beobachtet.

Das lytische Potential des Epithels, bestehend aus z.B. lysosomalen Hydrolasen, war erhöht, während die Aktivität von GGT und ATPase reduziert war.

Der Wundverschluß selbst war sehr langsam (ca. 10 Tage), die Adhäsion des neu gebildeten Epithels am darunter liegenden Stroma war sehr schlecht, es kam zur Bildung weißer Narben, die Tranparenz ging teilweise verloren, und zwar irreversibel.

Die Vorteile einer Behandlung mit PAI-2 bzw. mit Inhibitoren des Plasmin/Plasminogenaktivator-Systems liegen demzufolge darin, daß es nur auf diese Weise zu einer schnellen und voll zufriedenstellenden - voll transparent, keine Narben, gute Adhäsion, keine Neovaskularisierung, normale Stoffwechselaktivität der Hornhaut - Wiederherstellung der Hornhaut, auch auf histologischer Basis, kommt.

Tab. 4

| PLASMIN ACTIVITY IN THE TEAR FLUID ($\mu$g/ml) | | | |
|---|---|---|---|
| Corneal deep incision wound | | | |
| Time (h,d) | Buffer | r-PAI-2 | Aprotinin |
| 2 h | 0.2-0.4 | 0.2-0.4 | < 0.2 |
| 1 d | 0.4-1.0 | 0.2-0.4 | 0.2-0.4 |
| 4 d | 1.0-2.0 | 0.2-0.4 | 0.4-1.0 |
| 7 d | 1.0-2.0 | 0.2-0.4 | 0.4-1.0 |
| 14 d | 0.4-1.0 | 0.2-0.4 | 0.2-0.4 |
| 21 d | 0.2-0.4 | < 0.2 | < 0.2 |
| 28 d | < 0.2 | | |

Tab. 5     # Corneal deep incision wound
## of the rabbit eye

| Parameter | Test groups | | |
|---|---|---|---|
| | Buffer control | Aprotinin [60 μg/ml] | rPAI-2 [10 or 20 μg/ml] |
| **Tear fluid** | | | |
| - Plasmin activity | high increase | Reduced | nearly no increase |
| **Infiltration** | | | |
| - PMN´s | Numerous | Rare | Absent |
| **Epithelium** | | | |
| - Lysosomal Hydrolases | Increased | Low | Low |
| - GGT | Decreased | Normal | Normal ( early ) |
| - ATPase | Decreased | Normal | Normal ( early ) |
| **Wound healing** | | | |
| - Wound closure | Slowly ( 10 days ) | Normal | accelerated ( 4 days ) |
| - Epithelial adhesion to stroma | Bad | Acceptable | Very good |
| - Scar formation | + | - | - |
| - Transparency | Partially lost Irreversible | Transparent | Transparent |

**Beispiel 4:**

Verwendung von PAI-2 zur Behandlung nach Laser-Chirurgie

In dem von Lohmann et al. 1991 beschriebenen Verfahren wurde die photorefraktive Laserkeratektomie bei Kaninchen durchgeführt (193 nm excimer laser). Anschließend wurde PAI-2 in einer Konzentration von 20 μg/ml lokal ins Auge appliziert (zwei- bis dreimal täglich). Die Kontrollgruppe erhielt gleiche Volumina des Lösungsmittels (20 mM Glycin pH 7,2; 150 mM NaCl, 0,3 % Gelatinehydrolysat). Die Referenzgruppe erhielt entweder Aprotinin (5000 IU/ml; 2 mal täglich; 2 Wochen) oder 0,1 % Prednisolonacetat (2 mal täglich, 1 Woche; 1 mal täglich, 11 Wochen) oder 0,1 % Flurbiprofen (s. Prednisolonacetat).

Als Meßparameter dienten Ausbildung von Trübung, Plasminkonzentration in der Tränenflüssigkeit und der histologische Befund.

In der Kontrollgruppe stieg die Plasminkonzentration innerhalb kurzer Zeit von ca. 1 $\mu$g/ml auf Werte von bis zu 50 $\mu$g/ml an, normalisierte sich aber nach einer Woche wieder. Die Abschlußfläche zwischen dem neu gebildeten Epithel und dem verbliebenen Stroma war nicht glatt. Außerdem konnte neu gebildetes Matrixmaterial, z.B. Kollagen, nachgewiesen werden.

Im Stroma wurden vereinzelt Vakuolen beobachtet.

Ohne Behandlung besteht die Tendenz zur Regression zur Kurzsichtigkeit.

Als Folge der Bildung von Fehlstrukturen und von Neusynthesen war eine Trübung des Auges zu beobachten.

Bei Behandlung mit Prednisolonacetat wird im Prinzip das gleiche Ergebnis beobachtet, lediglich die Neusynthese von Kollagen ist etwas reduziert, wohingegen bei der Behandlung mit Flurbiprofen neben der Trübung eine signifikante Neusynthese von Kollagen zu beobachten ist.

Bei der Behandlung mit Aprotinin ist die Grenzfläche zwischen altem Stroma und dem neu gebildeten Epithel glatt. Dennoch wird eine durch Lichtreflexion bedingte Trübung der Hornhaut beobachtet. Es wurden keine Vakuolen beobachtet, auch waren die Basalzellen von normaler Größe. Jedoch wurde neues Kollagen synthetisiert.

Die Behandlung mit PAI-2 hat ein absolut klares, transparentes Auge zum Ergebnis (s. Tabelle 6). Selbst nach über drei Monaten wird keine Trübung - diese tritt normalerweise spätestens nach drei bis fünf Wochen auf - festgestellt. Das Epithel wird perfekt regeneriert:

die Grenzfläche zwischen altem Stroma und neu gebildtem Epithel ist glatt. Es gibt keine Ablagerungen, keine Vakuolen, keine Neusynthese von Kollagen und keine Hyperplasie des Epithels.

Eine Regression hin zur Kurzsichtigkeit wurde nicht beobachtet.

Selbst wenn man alle Referenzsubstanzen miteinander kombiniert ist das Behandlungsergebnis nicht besser als das der mit PAI-2 behandelten Gruppe.

Die Nachteile dieser Kombinationstherapie bzw. einer Therapie mit Referenzsubstanzen gegenüber einer Behandlung mit PAI-2 sind:

- Topische Applikation von Steroiden über drei Monate oder länger bringt ein unerwünschtes und hohes Risiko (abhängig von der Ablationsstärke) für die Bildung von Sekundärglaukomen.
- Aprotinin erwies sich als unverträglich mit Prednisolon
- Überempfindlichkeitsreaktionen gegenüber Aprotinin sind bekannt
- BSE-Problematik, Reinheitsgrad, Fremdprotein-Charakter sprechen gegen die Anwendung von Aprotinin.

Tab. 6

| THERAPIE NACH LASERKERATEKTOMIE: | | | | |
|---|---|---|---|---|
| | Behandlung Dauer (Monate) | Trübung | Epithelfläche | Kollagenneusynthese (=> Lichtreflexion) |
| Prednisolon | 3 | + | unregelmäßig | + |
| Flurbiprofen | 1 -3 | + | unregelmäßig | + + |
| Aprotinin | 0,5-1 | + | regelmäßig | + + |
| Aprot./Pred. Flurbiprofen | 3 | + | regelmäßig | -[1] |
| PAI-2 | 1 | - | regelmäßig | - |

[1] Aber Neusynthese einer noch unbekannten Substanz

## Patentansprüche

1. Verwendung von Inhibitoren von Plasminogenaktivatoren zur Herstellung eines Arzneimittels zur Therapie und/oder Prophylaxe von Entzündungen der Augen, der Ohren, der Haut, der Knochen, der Gelenke, des Darms oder anderer innerer Organe und/oder zur intra-und/oder postoperativen Behandlung und/oder zur Behandlung offener Wunden.

**2.** Verwendung nach Anspruch 1, dadurch gekennzeichnet, daß die Plasminogenaktivatoren Urokinase und/oder tPA sind.

**3.** Verwendung nach Anspruch 1, dadurch gekennzeichnet, daß die Inhibitoren PAI-1 und/oder PAI-2 sind, vorzugsweise Fragmente von PAI-2.

**4.** Verwendung nach Anspruch 1 zur Behandlung von Corneal ulcer, Uveitis, Konjunktivitis, Mittelohrentzündungen, Trommelfellentzündungen, Trommelfellriß mit entzündlichem Verlauf, Rötungen der Haut, Acantholyse, Pemphigus, Ekzem, Kontaktdermatitis, atopische Dermatitis, Verbrennungen mit entzündlichem Verlauf, Vaskulitis, dermale Ulcera, Osteoarthritis, IBD (Inflammatory Bowel Disease), Pankreatitis, Akne, Behandlung epithelialer Läsionen, Behandlung cornealer Läsionen, zur Behandlung der Narbenbildung bei Augenverletzungen, zur Behandlung von Skleritis, nicht-heilender Cornea-Erosionen, Xerose und/oder Keratose.

**5.** Verwendung nach Anspruch 1 zur intra- und/oder postoperativen Behandlung und/oder der Behandlung von offenen Wunden oder der genannten Entzündungen.

**6.** Verwendung nach Anspruch 1 zur Hemmung der Neovaskularisierung, zur Behandlung von cornealen Infiltraten der Augen oder von Hornhautentzündungen der Augen von Kontaktlinsenträgern.

**7.** Verwendung nach Anspruch 1, dadurch gekennzeichnet, daß die operative Behandlung eine Laserkeratektomie ist und/oder die offenen Wunden oder Entzündungen eine Folge der Laserkeratektomie sind.

**8.** Verwendung nach Anspruch 1 zur Behandlung von Augenentzündungen und Augentrübungen, z.B. nach Laserbehandlung, normaler Augenchirurgie (z.B. Vitrektomie, Kataraktchirurgie, extrakapsuläre Kataraktextraktion, Linsenoperationen, Linsenaustausch, Linsenimplantation, Keratoplastie, Hornhauttransplantationen) sowie als Konsequenz der folgenden Erkrankungen: Konjunktivitis, Keratokonjunktivitis, Keratokonjunktivitis sicca, Iritis, Iridocyclitis, Keratitis, Graves Ophthalmopathie, Mooren's ulcer, Vaskulitis, Uveitis, bei allergischen Erscheinungen im Auge, Infektionen, Stoffwechselstörungen, Entzündungserkrankungen und Autoimmunerkrankungen (z.B. Systemischem Lupus erythematodes, Wegener Syndrom, rheumatischer Arthritis, Sarcoidose, Polyarthritis, Pemphigus, Pemphigoid, Erythema multiforme, Sjögren's Syndrom, entzündliche Darmerkrankungen (Inflammatory Bowel Disease), Multiple Sklerose, Myasthenia gravis, Keratitis, Skleritis.

**9.** Verwendung nach Anspruch 1 zur Vermeidung der Narbenbildung am Auge nach chirurgischem Eingriff oder nach Verletzung.

**10.** Verwendung nach Anspruch 1 zur Hemmung der Neovaskularisierung bei Retinopathie, insbesondere bei Diabetikern, bei Retinaablösung, Gefäßverletzung der Retina oder Entzündung der Retina und Uvea oder bei Hornhauttransplantationen.

**11.** Verwendung nach Anspruch 1 zur Verwendung bei Transplantationen am Auge, insbesondere bei Hornhauttransplantationen.

**12.** Verwendung nach Anspruch 1 zur Behandlung von Ödemen im Augenbereich, z.B. Makula-Ödem, Submakula-Ödem, Ödem nach Photokoagulation, Cornea-Ödem, Konjunktiva-Ödem, Retina-Ödem, Ödem in der Umgebung des Auges.

**13.** Verwendung nach Anspruch 1 zur Behandlung von Infiltrationen im Augenbereich, insbesondere der Cornea, der Augenkammer, der Konjunktiva und der Sklera.

**14.** Verwendung nach Anspruch 1 zur Beschleunigung der Wundheilung, insbesondere im ophthalmologischen Bereich.

**15.** Verwendung nach Anspruch 1 zur lokalen Injektion, wie z.B. Injektion in Subkonjunktiva, Glaskörper, Augenkammer oder Sklera.

**16.** Verwendung nach Anspruch 1 zur Applikation mittels Mikroinjektion.

**17.** Verwendung nach Anspruch 1 zur Verwendung als Spüllösung.

**18.** Verwendung von Inhibitoren nach Anspruch 1 in Kombination mit einem biologisch abbaubaren Trägersystem.

**19.** Verwendung von Inhibitoren nach Anspruch 1 in Kombination mit einem pharmazeutisch akzeptablen Carriersystem, wie z.B. Cellulosederivaten, polymeren Matrices oder Kontaktlinsen.

**20.** Verwendung von Inhibitoren nach Anspruch 1 in Kombination mit Stabilisatoren.

**21.** Verwendung von Inhibitoren nach Anspruch 1 in Kombination mit weiteren Wirkstoffen.

**22.** Verwendung von Inhibitoren nach Anspruch 1, dadurch gekennzeichnet, daß als weitere Wirkstoffe ein oder mehrere Antibiotika verwendet werden.

| **EINSCHLÄGIGE DOKUMENTE** | | | EP 93105801.0 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| X | <u>EP - A2 - 0 356 945</u><br>(BEHRINGWERKE)<br> * Patentansprüche 1-3,6 *<br>-- | 1-3,5,<br>20 | A 61 K 37/64 |
| X | <u>WO - A1 - 91/09 124</u><br>(BIOTECH AUSTRALIA)<br> * Patentansprüche 23,24,27,<br> 28,31 *<br>-- | 1-3,<br>18,19 | |
| X | <u>EP - A2 - 0 451 130</u><br>(BALTIMORE BIOTECH)<br> * Patentanspruch 15; Seite<br> 10, Zeilen 4-23 *<br>---- | 1,2,4,<br>6,8 | |
| | | | **RECHERCHIERTE SACHGEBIETE (Int. Cl.⁵)**<br><br>A 61 K |

Der vorliegende Recherchenbericht wurde für alle Patentansprüche erstellt.

| Recherchenort | Abschlußdatum der Recherche | Prüfer |
|---|---|---|
| WIEN | 23-07-1993 | WOLF |